# EUROPEAN PATENT APPLICATION

(11) **EP 2 537 470 A1**
(43) Date of publication of application: **26.12.2012**
(21) Application number: 12170488.6
(22) Date of filing: 14.08.2007
(51) Int. Cl.: A61B 17/064, A61B 17/00, A61B 17/04, A61B 17/11, A61B 17/34, A61J 15/00

(54) **A tissue to tissue anchoring device and method of using the same**

(30) Priority: 28.08.2006 US 510974
(62) Divisional of application: 07826025.4
(71) Applicant: KIMBERLY-CLARK WORLDWIDE, INC., Neenah, WI 54956 (US)
(72) Inventor: Gobel, Fred, 69259 Wilhelmsfeld (DE)
(74) Representative: Davies, Christopher Robert

(57) **Abstract**

The invention describes a device (10) and method for anchoring two or more body tissue layers (22,24) to one another. The device (10) in one form may be a hollow, collapsible, microthin polymeric shaft (16) affixed to a noncollapsible tip (20) at a distal end (12) of the polymeric shaft (16). A preformed balloonable distention (18) formed in a discrete region of the shaft (16) proximal to the noncollapsible tip (20) would be adapted to anchor against one of the body tissue layers within a body orifice (26). A rod (50) attached at one end to the tip (20) which extends along the shaft (16) and terminates at a second end near a proximal end (14) of the device (10) would be used for transferring movement from the second end to the first end so as to effect a movement in the tip (20). The method pertains to the method of using such a device to secure one or more tissue layers together, ultimately to assist in the fusion of the layers.

## Description

### BACKGROUND OF THE INVENTION

The present invention relates to a device for anchoring a first body tissue to one or more additional body tissue layers. Such a device may be used to create and maintain an artificial stoma enabling access to a body cavity, such as used in the direct feeding of a patient's stomach. More particularly, the present invention may be used for percutaneously placing various gastric catheters, forming artificial stomas capable of accessing the gastrointestinal tract, and otherwise securing two or more tissue layers to one another until such time as a stoma is formed and the tissue layers fuse to one another.

It is recognized that numerous medical conditions exist in which it becomes necessary to gain percutaneous access to viscera such as the stomach or small intestines. Situations where a patient has lost the ability to swallow and will require long term nutritional support may dictate feeding directly into the stomach or jejunum. Feeding in this manner may be accomplished by inserting a feeding tube into the patient's stomach such that one end remains anchored in the stomach, while the other end remains external to the patient's body for connection to a nutrient source.

Such feeding tubes may be inserted into a patient's stomach in a number of ways. Feeding tubes may be endoscopically placed, surgically placed through an open incision, laparoscopically placed, or percutaneously placed under endoscopic, fluoroscopic or ultrasonic guidance. Different types of feeding tubes may be placed using these procedures, examples include gastrostomy, jejunostomy or gastro-jejunostomy. These tubes may be retained in the lumen (stomach or intestine) with a variety of retention anchors. These anchoring mechanisms include: inflatable balloons, obturatable domes, fixed dome-type bumpers, or suture wings.

Prior to placing the actual enteral feeding device, it has been preferred to perform a gastropexy procedure during placement. This procedure enables the physician to attach the visceral wall to the abdomen and to create the stoma tract through the two. This attachment is critical to prevent inadvertent separation and exposure of the peritoneal cavity to contamination and possible peritonitis.

Initial placement devices are often not readily removable without additional invasive surgical procedures. That is, many initially placed enteral catheters contain rigid retention members which cannot readily be passed through the stoma of the patient when it is desired to remove the initially placed device. Typically the t-shaped fastener or t-bar is not removable and is left in the body cavity where it is allowed to pass naturally in the patient's stool. In many cases the t-bar is not passed and remains within the body cavity. Moreover, during the six to eight weeks it takes for the fistula's stoma tract to be established, the anchoring mechanism of the prior art gastropexy device which typically consists of a small metal t-shaped fastener may embed itself into the gastric or intestinal wall and ultimately lead to infection. Furthermore, the t-bar itself may have sharp edges which can be uncomfortable for the patient.

In many of these procedures, in order to achieve the desired seal between the stomach and the abdominal wall, a traction force must be applied to the anchoring mechanism. The force is applied in such a way as to pull the stomach cavity to the abdominal wall so that the penetration through both may heal together thereby creating the passage or stoma leading from the patient's stomach, through the abdominal wall, to an external environment. It is necessary to apply this traction force for a period of a couple of days through a couple of weeks until the stoma site adequately heals. During this period the patient has reduced mobility which may lead to additional post-operative complications.

There is a need and desire for a device which may be used during initial placement or creation of a stoma site. Such a device would foster the permanent fusion of the stomach wall to the abdomen. This would serve to reduce the invasiveness of the procedure, greatly enhance wound healing, and enable immediate, post-placement gastric access for feeding and drainage, and ultimately allow atraumatic exchange of the low profile device. What is needed is a fixation device that is easy to place within an internal body cavity, allows for the formation of a stoma between the internal body cavity and the external environment, and enables the user to easily remove the fixation device when it is no longer necessary.

### SUMMARY OF THE INVENTION

In response to the foregoing problems and difficulties encountered by those of skill in the art, the present invention is directed toward an apparatus for insertion into a body orifice for anchoring a first body tissue layer to a second body tissue layer. In a first embodiment, a sheath having a longitudinal bore therethrough is provided. The sheath has a proximal end and a distal end, the distal end is adapted for insertion through at least two body tissue layers and into a body orifice from a point exterior to the body orifice. A hollow preshaped microthin polymeric device is used with the sheath. The device contains a shaft and a ballooning retention element located at or proximal to a distal end of the device. The device slidably engages the bore of the sheath such that the distal ends of each are proximate to one another. While they are engaged, the retention element is in a first collapsed state. A second free end of the device protrudes from the proximal end of the sheath. The device is adapted to be slid distally through the bore until at least the retention element is free of the sheath whereupon an inflation source may be applied to the device ballooning the retention element into a second expanded state.

Such an apparatus may utilize a device made wholly or partially of a polyurethane material. The sheath may be longitudinally splittable into two or more sections along a longitudinal separation line. Other embodiments may use a non-splittable sheath having a slot or groove at a distal end for the capture of the retention element therein. A retainer for affixing to a portion of the shaft protruding from the body to retain the apparatus in position may also be provided.

In another embodiment, an apparatus for insertion into a body orifice for anchoring a first body tissue layer to a second body tissue layer would have a hollow, collapsible, microthin polymeric shaft affixed to a noncollapsible tip at a distal end of the polymeric shaft. A preformed balloonable distention formed in a discrete region of the shaft proximal to the noncollapsible tip would be adapted to anchor against one of the body tissue layers within the body orifice. A rod may be attached at one end to the tip, allowed to extend along the shaft and terminate at a second end near a proximal end of the device. The rod would be adapted to transfer movement from the second end to the first end so as to effect a movement in the tip. The rod may be wholly or partially located internal to the shaft, external to the shaft, and/or within the shaft wall. This apparatus may have a proximally facing flattened surface on the balloonable distention located substantially normal to a longitudinal axis through the shaft.

A method for anchoring a first body tissue layer to a second body tissue layer would encompass the following steps: inserting a distal end of a longitudinally splittable sheath having a throughbore into a body through at least a first body tissue layer, a second body tissue layer, and into a body cavity, leaving a proximal end of the sheath protruding externally from the body; advancing a hollow preshaped microthin polymeric device having a shaft with a ballooning retention element integrated into a distal end of the shaft along the throughbore until the retention element protrudes from the distal end of the sheath; ballooning the retention member by inflating the member so that it expands from a first deflated condition to a second inflated condition; withdrawing the sheath from the body and sliding it free from the polymeric shaft at a proximal end of the shaft; and pulling the first and second body tissue layers one toward the other by applying a tensile force to the shaft so that the retention member contacts and draws one body tissue layer toward the other body tissue layer.

Another method may encompass the steps of perforating the first and second body tissue layers to create a stoma extending from a first region to a second region within a body orifice; advancing a hollow preshaped microthin polymeric device having a shaft with a ballooning retention element integrated into a distal end of the shaft into the body orifice by manipulating a rod attached at the distal end and extending to a proximal end until the ballooning retention element is situated; ballooning the retention member by inflating the member so that it expands from a first deflated condition to a second inflated condition; and pulling the first and second body tissue layers one toward the other by applying a tensile force to the shaft so that the retention member contacts and draws one body tissue layer toward the other body tissue layer.

Additional steps may include by itself or in any combination, the following: tying off an end of the shaft which protrudes externally from the stoma; engaging an end of the shaft which protrudes externally from the stoma with a thin retainer adapted to secure the protruding shaft proximal to the perforation; and/or bandaging the protruding shaft and retainer.

The apparatus and methods described herein would be suitable for use in performing a gastropexy procedure wherein one of the body tissue layers comprises the abdominal wall and the other layer comprises the stomach. Other objects, advantages and applications of the present invention will be made clear by the following detailed description of a preferred embodiment of the invention and the accompanying drawings wherein reference numerals refer to like or equivalent structures.

### BRIEF DESCRIPTION OF THE DRAWINGS

FIG. 1 depicts an illustrative view of one embodiment of the present invention.
FIG. 2 depicts an illustrative view of the FIG. 1 embodiment secured in place.
FIG. 3 depicts an illustrative view of a first embodiment of a retainer for use with the FIG. 1 device.
FIG. 4 depicts an illustrative view of an alternative embodiment of retainer for use with the FIG. 1 device.
FIG. 5 depicts an illustrative view of a second embodiment of the FIG. 1 device.
FIG. 6 depicts an illustrative view of a first embodiment of a sheath for use with the FIG. 1 device.
FIG. 7 depicts an illustrative view of an alternative embodiment of a sheath for use with the FIG. 1 device.

### DETAILED DESCRIPTION OF THE PRESENT INVENTION

In response to the foregoing challenges that have been experienced by those of skill in the art, the present invention is directed toward an apparatus for anchoring a first body tissue to a second body tissue and a method for using such an apparatus.

Should such a device be caused to enter a body from an exterior position, traverse two or more body tissue layers, and be situated at a distal end within a suitable body cavity or orifice, the device may be used to pull the two or more tissue layers into contact thereby anchoring one to the other. Such a device would prove useful in the formation of artificial stomas into or within a living body.

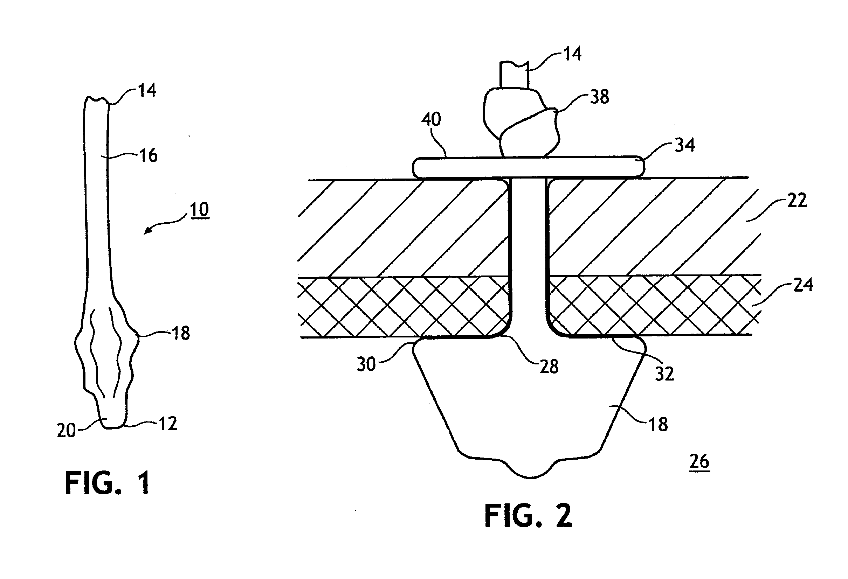

FIG. 1 shows a first embodiment of an inventive apparatus for insertion into a body orifice or cavity for anchoring a first body tissue layer to at least one other body tissue layer. In one simple embodiment, a device 10 is provided; the device 10 has a distal end 12 and a proximal end 14. As used herein, distal refers generally to the direction of the patient, while proximal refers to the direction of the user. The device 10 is formed of a biocompatible polymeric material configured as a hollow shaft 16 with a ballooning retention element 18 at or near the distal end 12.

According to some embodiments the material selected to form the device 10 may include polyurethane (PU) , low-density polyethylene (LDPE), polyvinyl chloride (PVC), polyamid (PA) or polyethylene teraphthalate (PETP), These materials are biocompatible and, when being processed into correspondingly thin walls, are especially suited for forming the ballooning retention element 18. Copolymer admixtures for modifying the characteristics of the material are also possible, for example a low density polyethylene and ethylenevinylacetate copolymer (LDPE-EVA), or blends of the above mentioned materials (e.g. PU with PVC or PU with PA) would be considered suitable for such a device. Other materials would also be suitable so long as they exhibit properties enabling them to be processed into devices having microthin walls, and do not deform elastically to a degree that an anchoring function of the balloon element can not be secured respectively that the balloon element might slip through the insertion channel in the body wall.

Formation of the ballooning retention element 18 may be achieved by situating the shaft 16 at an appropriate position in a suitable mold (not shown), applying heat and expanding the heated region of the shaft itself controllably, typically by inflating the region within the heated mold itself. This process enables the discrete region to be distended without otherwise damaging the shaft. Due to the controlled distention of the region forming the ballooning retention element, the wall thickness is characteristically reduced in that area. Stretching the region during this process serves to molecularly align the polymeric chains thus making the product otherwise stronger than it would be even at microthin wall thicknesses. Such techniques would be known and understood by those of skill in the art.

Final wall thicknesses for the ballooning retention element 18 are considered to be microthin in nature, and may range from about 25 microns down to about 3 microns whereas the shaft wall thicknesses may range from about 50 microns to about 150 microns. As seen in FIG. 1, the ballooning retention element 18 is not elastically distendable but is preformed and exists in a collapsed condition when not inflated.

During the manufacturing process for the device, the distal end 12 would be blocked, sealed, or otherwise made fluid tight. Although the distended region or ballooning retention element 18 may be situated at the distal end 12, it may alternatively be proximal to the distal end such that the device 10 at the distal end 12 terminates in a nipple or tip 20. This tip 20 may also be made non-collapsible by the filling of the tip 20 with a potting compound such as a polymer, for example, silicone or the like, or another biocompatible material. This would provide a degree of rigidity to the distal end 12 of the device 10 and may be desirable in some embodiments.

Turning now to FIG. 2, the device 10 may be seen in use to secure a first body tissue layer 22 to a second body tissue layer 24. The ballooning retention element 18 is situated within a body orifice 26 and is depicted in an inflated condition. The proximal end 14 of the device 10 is located at a position external to the orifice 26, in many cases external to the body itself.

During the molding process, additional desirable features may be incorporated into the ballooning retention element which would prove useful in the application of the invention. For example, the ballooning retention element 18 may be preshaped so as to possess sufficiently small shoulder radii at regions 28 and 30 so that a face 32 may be created which is flat in shape. This would create a large resting or bearing surface to seat with the second body tissue layer 24. The surface area of the face 32 working in conjunction with inflation of the ballooning retention element 18 would help minimize the likelihood of the retention element slipping out of the orifice 26.

Other desirable retention element shapes may be created as well depending upon the application. For example, the overall geometry of the ballooning retention element 18 may be bullet-shaped, disc-shaped, spherical, cylindrical, frustoconical or any other suitable shape limited only by the purpose intended and the skill of those in the art at forming preshaped balloons.

Once the device 10 is in place, the ballooning retention element 18 properly situated and inflated, as described below in more detail, in many embodiments the proximal end may simply be tied off. The reason that tying the device at the proximal end would be possible is due to the small size of the device and the low inflation pressures needed to fully inflate the device once it is in place. It is envisioned that the diameter of the shaft 16 in many embodiments may be as small as from about 0.8 mm to about to 1.5 mm, and the device may be considered fully inflated at pressures as low as from about 50 mbar to about 200 mbar.

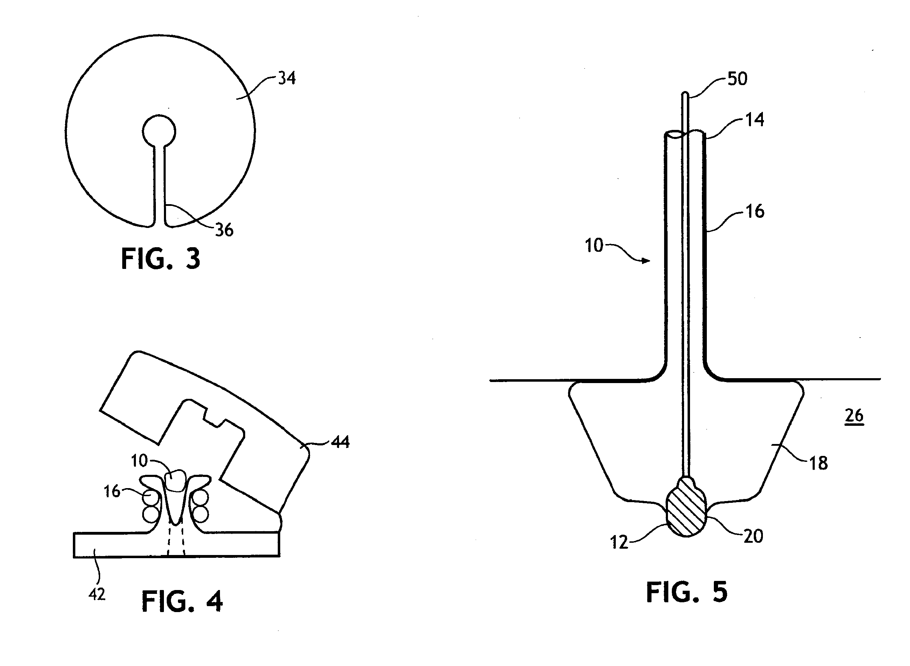

In the above embodiments as well as others, a retainer 34 may be provided so as to retain the device 10 in position. The retainer 34 is envisioned to have numerous possible configurations some of which will be discussed at greater length in this specification. In a first embodiment, depicted in FIG. 3, the retainer 34 may be configured as a simple disc, button, or ring. This retainer 34 could be provided with a through-hole or slot 36 for capturing a knot 38 formed by tying off the shaft 16. The knot 38 would seat against an exterior facing surface 40 of the retainer 34 as shown in FIG. 2. One advantage gained by the use of such a retainer is that the retainer could be made as thin as possible, on the order of 1 to 2 mm, and in some cases dependent upon the material from which it is manufactured, even thinner.

A retainer of this construction would have a very low profile and could easily be concealed by the application of a bandage over the skin of the patient. This would enable the device 10 to be in place, performing its function, yet not be noticeable to the public. This may provide a beneficial effect to the health and mental well-being of the patient as well as enable the patient to be more active in that little of the device would protrude from the patient's body. Moreover, this would assist in maintaining sterility of the site, and may minimize the potential for inadvertent traumatic injury to the area.

In a second embodiment depicted in FIG. 4, a multicomponent design may be used to secure the device in place. One example may utilize a base plate 42 for seating against the patient's body, and a lid or cap 44 secured to the cap to cover the base plate 42 as well as that portion of the device 10 protruding from the body. The base plate 42 may be configured similarly to the FIG. 3 retainer in that it would possess a slot or central opening which would enable the base plate to secure the shaft 16. The shaft 16 may be pulled under tension and wedged or otherwise secured into the base plate, for example, by engaging the shaft 16 with a fixture 46 such as by passing the device 10 through the fixture 46 and wrapping the shaft 16 around the device to secure similarly to a cleat. This configuration may be covered with a bandage as described above, or the cap 44 may be secured over the base plate. The cap and base plate configuration may also be made to have a low profile. Such an arrangement may range in thickness from about 5 mm to about 15 mm in dimension as measured normal to skin surface of the patient.

A third embodiment, may be similar to that depicted in US Patent Application Serial Number 11/139,927 filed on May 27, 2005 entitled "Clamp for Flexible Tube" which is copending and commonly assigned, the disclosure of which is herein incorporated by reference in its entirety.

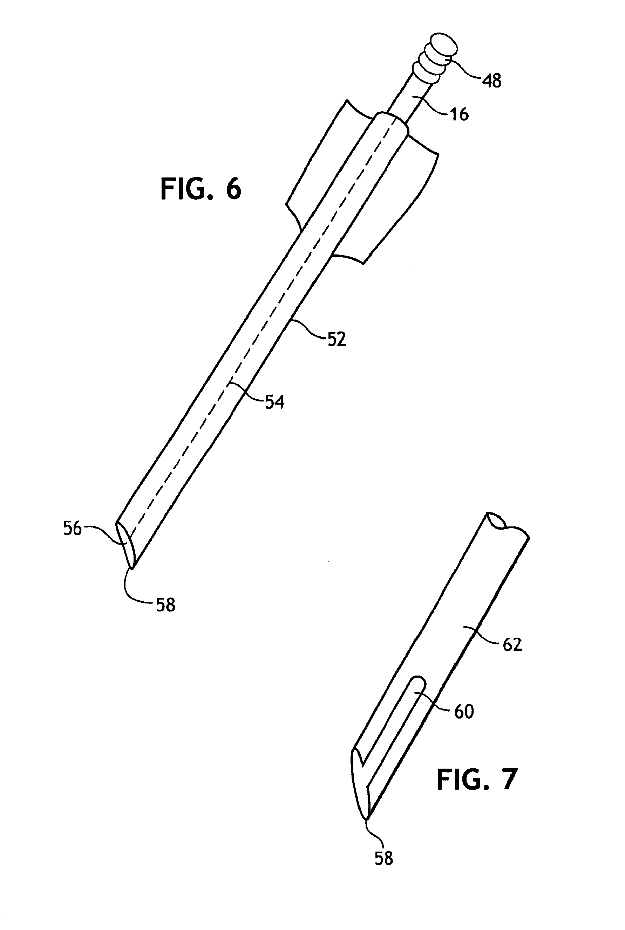

To inflate the device, a connector 48, depicted later in FIG. 6, may be situated at or near the proximal end 14 of the device 10. The connector 48 would be capable of engaging an inflation source (not depicted) which due to the small size of the device 10 itself, may simply be a syringe capable of injecting a fluid into the device 10. As such, suitable connectors may include luer fittings and the like and are known and understood by those of skill in the art.

In some embodiments, the connector 48 may comprise a releasable one-way valve disposed at the proximal end of the device 10. Appropriate valves capable of serving in this function are known and their incorporation into the device 10 would prevent inadvertent deflation once the inflation source was removed from the connector. Such devices are well known in the medical field and would be understood by those having skill in the art. These valves are suitable for actuation by means of the syringe. It would be understood that such a valve would serve as a means to control the injection of fluids into or the removal of the same from the device 10. As would be apparent, control of the inflation of the device 10 enables the user or a physician, etc., to selectively control inflation and deflation of the ballooning retention element 18.

A number of techniques and adaptations to the basic device may be utilized to initially situate the device 10 within the body orifice 26. They should be considered as adding optional structure to the basic device described above. Each of these new structures may be substituted in whole or in part in any combination for any other to create additional embodiments. For example, one optional structure may be a rod 50. One such example is depicted in FIG. 5. The rod 50 would serve as a rigid or semi-rigid linkage or connection between the nipple or tip 20 and a point exterior to the orifice 26 and would be adapted to be physically grasped or manipulated by a clinician.

In some embodiments, such as that shown in FIG. 5, the rod 50 may comprise any number of rigid or semi-rigid constructs, including a wire, shaft, tube, or thin bar which is embedded at a distal end into a potting compound contained within the tip 20. In any event, the rod 50 would be provided with an end proximal to the tip which would be accessible to a clinician even should the device be situated within the patient. The rod itself may be situated internal to the shaft 16 and as such, would extend along its length. In such embodiments, the rod 50 would be sized so as not to completely occlude the inflation and deflation features of the ballooning element 18. In some embodiments, the rod 50 would be sized such that its cross sectional area was between about one-third to two-thirds of the cross sectional area of the inside diameter of the shaft.

The rod 50 would terminate at a point within the potting compound and be bedded therein as described above. Though the potting compound would be situated within an internal portion of the device and thus would not normally be accessible to the body, in most instances it likely would comprise a biocompatible material such as the silicone plug as described earlier. Whatever material is selected for use, it should be capable of capturing one end of the rod. In forming this linkage or connection, it would be understood that any force applied to the one end of the rod is transferred to the other end without buckling. The clinician by manipulating the rod could effect the position of the tip within the orifice. In those embodiments having a rod 50, the rod may be removable or sufficiently flexible to enable the shaft 16 to be tied off as described above. If the rod 50 were wire-like, in some cases it may remain in place and not interfere with the tying process and may even prove useful in assisting with the tying of the shaft so as to be fluid tight, however, this certainly should not be considered to constitute a requirement.

Though the embodiment depicted and described places the rod 50 within the device 10 and shaft 16, this is not a requirement for any of the embodiments. In fact, the rod may run along an exterior surface of the device or be placed within a wall of the device itself. In any event, it should not be lost sight of that the purpose of the rod is to enable placement of the device itself into, the patient and allow manipulation of the tip 20, which is located at the distal end 12 of the device. By articulating or otherwise moving the rod 50 at or near the proximal end 14 of the device, which would be external to the patient once the device is in place, the articulation is transferred through the device 10 from the proximal end 14 to the distal end 12.

The above described embodiments may be further adapted by additional elements to create further embodiments. Additional embodiments contemplate the use of more refined introducers for placing the device 10 within a patient. For example, FIG. 6 depicts a splittable sheath 52 which is longitudinally splittable along a separation line 54. The separation line is formed by etching or perforating the sheath longitudinally along its axis. The sheath 52 has a longitudinal bore 56 therethrough within which the device 10 resides and is subsequently deployed. The device 10 itself, may be fed into the bore 56, and in many embodiments may be preloaded during the manufacturing process.

The sheath 52 may be provided with a trocar tip 58 capable of creating the initial penetration through the tissue layers. The device 10 should be capable of deployment and inflation without risk of puncture or damage. This is especially of concern in those embodiments having the trocar tip 58. The rod 50 as depicted in FIG. 5 may prove useful in such embodiments in that it may be used to push the ballooning retention element 18 out of the sheath 52 prior to inflation. As such, it would be simple to ensure that the ballooning retention element 18 be located at a puncture safe distance from the trocar tip 58 yet be placed close to its ultimate location. At that time the sheath 52 may be removed from the body and the ballooning retention element 18 inflated.

As stated, in the FIG. 6 embodiment, the sheath 52 is designed to longitudinally split along its length into at least two halves which may be removed from around the shaft 16 without requiring any inadvertent displacement of the device 10. This feature may be found to be useful for those embodiments in which the proximal end 14 of the device 10 has been fitted with a connector 48 which would otherwise interfere with the sheath 52 in sliding over the device 10 during removal.

An alternative to the fully splittable sheath 52 of FIG. 6 may be seen in the FIG. 7 embodiment in which a groove or slot 60 is machined into a sheath 62. Such a slot 60 may be adapted to engage a portion of the ballooning retention element 18 or even the rod 50 if the embodiment possessed one. Once the device is in place, the sheath 62 may be withdrawn from the patient.

In either described embodiment, it may be seen that the sheath 52 or 62 may serve as the rigid or semi-rigid implement for insertion of the device 10 within the body. Of course, the rod 50 may still prove useful in certain embodiments such as those described above in which it is desirable to deploy the device from the bore of either sheath. Another feature that may be incorporated into any of the embodiments is to provide the device 10 with a lengthening feature. This may prove additionally useful in those embodiments which are deployed via a rigid or semi-rigid sheath yet not require the need for the rod 50 to deploy the device 10. In such an embodiment, as the ballooning retention element 18 is inflated, inflation is first caused to extend the device longitudinally prior to any radial expansion of the ballooning retention element 18. Such a feature would enable the inflation process itself to deploy the ballooning retention element from the sheath.

Once the ballooning retention element 18 had fully deployed from the sheath 52 or 62 and the likelihood of damage to the device 10 is minimized, the sheath may be withdrawn from the body in any of the fashions described above and the ballooning retention element may continue to be inflated sufficiently so as to secure the device. This controlled expansion may be accomplished by molding the ballooning retention element in a manner that will specifically cause it to deploy from the sheath, or by preloading the device within the sheath so that it will do the same. One possible technique which may be used is to preload the sheath with the device, but to twist the device torsionally during the loading process and bunch up a portion of the device within the sheath. The twist would occlude the passage of the inflation fluid but would cause the device to move until such time as the twist were to clear the sheath. At that time, the device would untwist allowing the ballooning retention element to expand. Obviously folding the device without twisting may be made to accomplish the same effect.

Throughout the specification, the device has been described as being inflated with air from a syringe, however it should be understood that the device may be inflated and deflated upon application or removal of a fluid source such as water or saline, in addition to air. Other fluids, both gaseous and liquid, may also be used and would be understood by those of skill in the art.

Due to the controllable collapsibility of the device 10 it would be more amenable to atraumatic removal from the stoma than are prior art devices. This is because the present invention does not require the significant trans-abdominal exertion typically associated with those prior art devices containing a rigid shaft for carrying the balloon component. In the prior art devices, the mechanics of the balloon member are typically altered negatively over time, for example, balloon members associated with the prior art are known to stiffen and lose their ability to retract fully into the shaft completely. This results in the creation of traumatizing folds that may exacerbate healing of the stoma site upon removal or subsequent manipulation of the catheter. Proper selection of materials will prevent the present invention from exhibiting such features.

The device 10 may be positioned in the living body using techniques and procedures known and understood by those of skill in the art. For example, were the device to be used to anchor an anterior wall of a patient's stomach to the patient's abdominal wall prior to the placement of an enteral feeding device, the patient would be made to lie in a supine position. The patient's gastric lumen would be insufflated via a previously positioned nasogastric tube until it was sufficiently distended. Local anesthesia would be applied to the patient's abdomen and a puncture needle would be plunged through the patient's abdominal wall and into the gastric lumen. Aspiration of air from the needle or puncture site would signify proper needle placement.

In those embodiments that do not require the use of a sheath, the device may be inserted directly through the puncture site by manipulation of the rod as earlier described. In those embodiments with a sheath, the situation would be similar, i.e., the sheath would be inserted through the puncture site and the device deployed through it. Of course those sheaths having a trocar tip would also serve as the puncture needle. In any event, once the device 10 was in place, the ballooning retention element 18 would be inflated. The clinician would exert a gentle tractive force on the protruding shaft until the anterior wall of the stomach contacts the anterior abdominal wall. At that point the retainer would be slid over the shaft and the shaft would be tied in place or would otherwise be engaged with the retainer. In those embodiments where a multipart retainer was used, the shaft would be engaged with the base plate. The cap, if available, would be placed over the base plate.

In many procedures, a plurality of devices are used in close proximity to one another. For example, in a gastropexy procedure, often three or four devices are used in conjunction with one another. Once the stomach wall and the abdominal wall are secured to one another, a gastrostomy tube is often placed into the stomach lumen by making an additional incision at a location interior to the perimeter of the plurality of gastropexy devices. In any event, an individual retainer may be made to have the capability of securing more than one device 10 therein. That is, a single retainer may be used to secure two or more of the devices described above, so long as the devices were sufficiently closely spaced to one another.

As used herein and in the claims, the term "comprising" is inclusive or open-ended and does not exclude additional unrecited elements, compositional components, or method steps.

While various patents have been incorporated herein by reference, to the extent there is any inconsistency between incorporated material and that of the written specification, the written specification shall control. In addition, while the invention has been described in detail with respect to specific embodiments thereof, it will be apparent to those skilled in the art that various alterations, modifications and other changes may be made to the invention without departing from the spirit and scope of the present invention. It is therefore intended that the claims cover all such modifications, alterations and other changes encompassed by the appended claims.

## Claims

1. An apparatus for insertion into a body orifice for anchoring a first body tissue layer to a second body tissue layer comprising:
a hollow, collapsible, microthin polymeric shaft affixed to a noncollapsible tip at a distal end of the polymeric shaft, a preformed balloonable distention formed in a discrete region of the shaft proximal to the noncollapsible tip adapted to anchor against one of the body tissue layers within the body orifice;
a rod attached at one end to the tip, extending along the shaft and terminating at a second end near a proximal end of the device, the rod adapted to transfer movement from the second end to the first end so as to effect a movement in the tip.

2. The apparatus of claim 1 comprising a proximally facing flattened surface on the balloonable distention located substantially normal to a longitudinal axis through the shaft.

3. The apparatus of any preceding claim wherein the rod extends through an interior space within the shaft.

4. The apparatus of any preceding claim comprising a flattened, substantially washer-like retainer.

5. The apparatus of any preceding claim comprising a base plate for capturing a portion of the shaft and retaining it therein.

6. An apparatus for insertion into a body orifice for anchoring a first body tissue layer to a second body tissue layer comprising:
a sheath having a bore therethrough, a proximal end, and a distal end for insertion through at least two body tissue layers and into a body orifice from a point exterior to the body orifice;
a hollow preshaped microthin polymeric device having a shaft and a ballooning retention element proximal to a distal end, the device being slidably engaged within the bore of the sheath such that the distal ends of each are proximate to one another and the retention element is in a first collapsed state and a second free end of the device protrudes from the proximal end of the sheath, the device adapted to be slid distally through the bore until at least the retention element is free of the sheath whereupon an inflation source may be applied to the device ballooning the retention element into a second expanded state.

7. The apparatus of claim 6 wherein the device comprises a polyurethane material.

8. The apparatus of claim 6 wherein the sheath is splittable into two or more sections along a longitudinal separation line.

9. The apparatus of claim 6 wherein the sheath comprises an open-ended slot for capturing and retaining the ballooning retention element prior to slidable deployment of the ballooning retention element.

10. The apparatus of claim 6 wherein the bore comprises a diameter of less than about 21 gauge.

11. The apparatus of claim 6 wherein the retention element in an inflated state comprises a face for contacting and resting against one of the surfaces.

12. The apparatus of claim 6 comprising a retainer for affixing to a portion of the shaft protruding from the body to retain the apparatus in position.

13. The apparatus of claim 6 comprising a retainer for securing the device in place.

14. The apparatus of any preceding claim for performing a gastropexy procedure.

15. The apparatus of any preceding claim wherein the retention element is preformed and not elastically distendable.
